# EUROPEAN PATENT APPLICATION

(11) **EP 4 813 311 A1**
(43) Date of publication of application: **30.09.2026**
(21) Application number: 25166930.5
(22) Date of filing: 28.03.2025
(51) Int. Cl.: A61B 6/12, A61B 6/00, A61B 6/46, A61B 90/00

(54) **INTRALUMINAL DEVICE GUIDANCE WITH MINIMIZED X-RAY DOSE**

(71) Applicant: Koninklijke Philips N.V., 5656 AE Eindhoven (NL)
(72) Inventor: VAN DER MAREL, Kajo, Eindhoven (NL); HENDRIKS, Bernardus Hendrikus Wilhelmus, Eindhoven (NL); VERSTEGE, Marco, Eindhoven (NL); TERMEER, Maurice Alain, Eindhoven (NL); HOMAN, Robert Johannes Frederik, Eindhoven (NL); VAN GELOOF, Marinus Wilhelmus, Eindhoven (NL); LOKKERBOL, Coenraad Christiaan Albert, Eindhoven (NL); KREMER, Frans Henk, Eindhoven (NL); ADMIRAAL, Alexander Johannes, Eindhoven (NL); ELENBAAS, Thijs, Eindhoven (NL)
(74) Representative: Philips Intellectual Property & Standards

(57) **Abstract**

The present invention relates to Intraluminal device guidance with minimized X-ray dose. In order to provide further dose reduction while solving the conflict of objectives to not impair guidance during intravascular interventions, a control device (10) for intraluminal device guidance with minimized X-ray dose is provided. The device comprises a data input 12, a data processor 14 and an output interface 16. The data input is configured: to provide an X-ray image (18) of a vascular structure with an inserted device having at least one distinctive feature identifiable in the X-ray image. The X-ray image is acquired with a primary radiation characteristic. The data processor is configured: to determine a signal to noise ratio relating to an image portion comprising the at least one distinctive feature; to compute a secondary radiation characteristic for acquiring secondary X-ray images with the secondary radiation characteristic to generate images for further guidance of the inserted device; and to select the primary radiation characteristic or the secondary radiation characteristic for acquiring further X-ray images. The selecting is dependent on at least one of the parameters comprising: current location within subject and current treatment phase. The output interface is configured: to provide the selected radiation characteristic for acquiring further X-ray images.

## Description

### FIELD OF THE INVENTION

The present invention relates to a control device for intraluminal device guidance with minimized X-ray dose, to a medical X-ray imaging system for intraluminal device guidance and to a method for intraluminal device guidance with minimized X-ray dose.

### BACKGROUND OF THE INVENTION

During e.g. intravascular interventions, guidance is required to steer and operate instruments along the vascular structure, i.e. inside the vessels. Medical imaging like X-ray radiation is used for providing visual guiding support to a clinician handling an interventional instrument like a catheter. Since X-ray radiation is harmful in high dose to a subject as well as to staff members, it is generally desired to avoid unnecessary radiation. Even though detectors with higher sensitivity are provided allowing to use less dose, it has been shown that further dose reduction is in conflict with guidance requirements.

### SUMMARY OF THE INVENTION

There may thus be a need to provide further dose reduction while solving the conflict of objectives to not impair guidance during intravascular interventions.

The object of the present invention is solved by the subject-matter of the independent claims; further embodiments are incorporated in the dependent claims. It should be noted that the following described aspects of the invention apply also for the control device, for the medical X-ray imaging system and for the method for intraluminal device guidance with minimized X-ray dose.

According to the present invention, a control device for intraluminal device guidance with minimized X-ray dose is provided. The device comprises a data input, a data processor and an output interface. The data input is configured to provide an X-ray image of a vascular structure with an inserted device having at least one distinctive feature identifiable in the X-ray image. The X-ray image is acquired with a primary radiation characteristic. The data processor is configured to determine a signal to noise ratio relating to an image portion comprising the at least one distinctive feature, and to compute a secondary radiation characteristic for acquiring secondary X-ray images with the secondary radiation characteristic to generate images for further guidance of the inserted device, and to select the primary radiation characteristic or the secondary radiation characteristic for acquiring further X-ray images. The selecting is dependent on at least one of the parameters comprising current location within the subject and the current treatment phase. The output interface is configured to provide the selected radiation characteristic for acquiring further X-ray images.

As a result, the issue is solved to minimize X-ray radiation dose while still being able to guide the instruments to their intended location inside the body.

For finding the at least one distinctive feature, the device is identified first, and then a distal end is identified by moving along the device in the image data.

The adaptation of the radiation dose is purely image-based.

The current location relates to an actual anatomic situation. The current treatment phase relates to an actual procedure performed for treating the subject.

According to an example, the distinctive feature of the device is at least one of the group comprising: marker of the device, tool of the device and tip of the device.

According to an example, for the computing of the secondary radiation characteristic, the data processor is configured to choose a decreased radiation dose that results in a lower signal to noise ratio in the image portion comprising the at least one distinctive feature, which lower signal to noise ratio is above a predetermined threshold dependent on the distinctive feature.

This ensures that an adapted compromise is found considering the opposite targets of low dose and good visibility.

According to an example, for the computing of the secondary radiation characteristic, the data processor is configured to assume a navigation path based on the device inside the vessel, and to assess an upcoming image area ahead for adapting the radiation dose for an upcoming imaging step. As an effect, imaging dose is used to optimum effect, since upcoming structures can be taken into account.

According to an example, for determining the signal to noise ratio of an image, the data processor is configured to identify relevant structures in the image, to determine the average pixel intensity for these structures, to measure noise as the standard deviation of pixel intensities in a background region without significant signal, and to determine the signal to noise ratio by dividing the average signal by the standard deviation of the noise.

According to an example, for determining whether the inserted device is within the predetermined region of the subject, the data input is configured to provide location information of the predetermined region of the subject. The data processor is configured to register the location information to a current X-ray image, to identify the location of the device in the current X-ray image, and to determine a spatial relation of the location of the device and the predetermined region of the subject.

According to an example, for identifying the distinctive feature in the X-ray image, the data processor is configured to identify at least one of the group comprising i) instrument classification, ii) device-related data and iii) type of treatment.

According to an example, for providing location information, the data input is configured to provide pre-operational image data or image data acquired during the current intervention.

According to an example, the data processor is further configured to determine whether the inserted device is within a predetermined region of the subject, and to select to acquire X-ray images with i) the secondary radiation characteristic if the inserted device is not within the predetermined region of interest, or with ii) the primary radiation characteristic if the inserted device is within the predetermined region of interest.

According to the present invention, also a medical X-ray imaging system for intraluminal device guidance is provided. The system comprises an X-ray imaging device and a control device according to one of the preceding examples. The X-ray imaging device is configured to provide the X-ray image of the vascular structure with an inserted device having at least one distinctive feature identifiable in the X-ray image. The control device is configured to provide the selected radiation characteristic to the X-ray imaging device to acquire further X-ray images.

According to an example, the control device is configured to provide the secondary radiation characteristic to the X-ray imaging device to acquire secondary X-ray image data with the secondary radiation characteristic.

According to the present invention, also a method for intraluminal device guidance with minimized X-ray dose is provided. The method comprises the following steps:
- Providing an X-ray image of a vascular structure with an inserted device having at least one distinctive feature identifiable in the X-ray image; the X-ray image is acquired with a primary radiation characteristic;
- Determining a signal to noise ratio relating to an image portion comprising the at least one distinctive feature;
- Computing a secondary radiation characteristic for acquiring secondary X-ray images with the secondary radiation characteristic to generate images for further guidance of the inserted device; and
- Selecting the primary radiation characteristic or the secondary radiation characteristic for acquiring further X-ray images; the selecting is dependent on at least one of the parameters comprising: current location within subject and current treatment phase.

According to an aspect, instruments are guided to a treatment location inside the body by using X-ray imaging. However, the X-ray radiation dose is minimized while still being able to guide the instruments to their intended location inside the body.

According to an aspect, instruments with incorporated markers are used to enable better visualization under X-ray guidance. This better visualization is used to reduce the X-ray usage during advancing the instrument. Initially, a first X-ray acquisition of the instrument with markers is provided, and the signal to noise ratio of the markers is determined. Then, subsequent X-ray images are taken with a respectively calculated lower dose, dependent on the current image situation and its signal to noise ratio.

In an option, the dose lowering is applied when an instrument is not in a region of interest like a treatment area. The region of interest or treatment area can be determined by a pre-op image or other image available in the cathlab environment. Another option is to use a first X-ray image of the instrument to determine whether it is in a region of interest, like a treatment area, or not: if not, lower dose is applied for subsequent images until instrument is in the region of interest, e.g. the treatment area; when in a region of interest / treatment area, normal settings are used. Thus, an ultra-low dose, or at least minimized dose device guidance with X-ray is provided.

An example for application of the present invention is the field of navigation of instruments under X-ray guidance to a treatment area.

An example of application are neuro-interventional therapies, e.g. brain aneurysms, strokes and the like, where the X-ray dose to the abdominal area can be reduced until guidewire and (micro)catheter are in place, and until therapy device, such as thrombectomy devices, balloons, stents, arrive at the cranial treatment area. Another example of application are percutaneous coronary interventions where markers on e.g. PTA catheters can be followed with low X-ray radiation dose until the cardiac region is reached.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a control device for intraluminal device guidance with minimized X-ray dose.
Fig. 2 shows an example of an X-ray image of a subject with an inserted instrument.
Fig. 3 shows an example of a medical X-ray imaging system for intraluminal device guidance.
Fig. 4 shows basic steps of an example of a method for intraluminal device guidance with minimized X-ray dose.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of", when preceding a list of elements, modify the entire list of elements and do not modify the individual elements of the list.

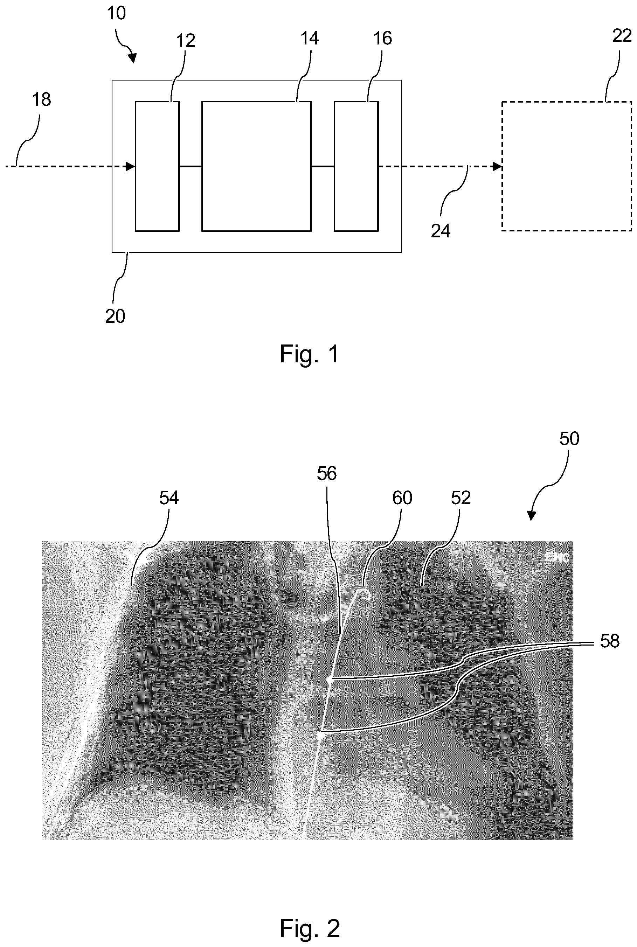

Fig. 1 schematically shows an example of a control device 10 for intraluminal device guidance with minimized X-ray dose. The control device 10 comprises a data input 12, a data processor 14 and an output interface 16. The data input 12 is configured to provide an X-ray image 18 of a vascular structure with an inserted device having at least one distinctive feature identifiable in the X-ray image. The X-ray image 18 is acquired with a primary radiation characteristic. The data processor 14 is configured to determine a signal to noise ratio relating to an image portion comprising the at least one distinctive feature. The data processor 14 is also configured to compute a secondary radiation characteristic for acquiring secondary X-ray images with the secondary radiation characteristic to generate images for further guidance of the inserted device. The data processor 14 is further configured to select the primary radiation characteristic or the secondary radiation characteristic for acquiring further X-ray images. The selecting is dependent on at least one of the parameters comprising: current location within subject and current treatment phase. The output interface 16 is configured to provide the selected radiation characteristic for acquiring further X-ray images.

A frame 20 indicates a common enclosure or supporting structure such as a housing. The data input 12, the data processing arrangement 14 and the output interface 16 can be arranged in an integrated way. However, in another option, the data input 12, the data processing arrangement 14 and the output interface 16 are provided separately.

The term "data input" relates to providing or supplying data for data processing steps. The data input can also be referred to as image data input. The data input 12 can also be referred to as data supply, as image data supply, as image input, as input unit or simply as input. In an example, the image data input 12 is data-connectable to an imaging source arrangement.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input 12 and the output interface 16.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the controller can be provided by the output interface 16.

As an option, the device 10 further comprises a display 22 configured to present the X-ray image data acquired with the selected radiation characteristic for acquiring further X-ray images. As an option, the display 22 is also configured to display the X-ray image 18 of a vascular structure with an inserted device having at least one distinctive feature identifiable in the X-ray image provided to determine the signal to noise ratio.

An arrow 24 indicates a data-connection of the output interface 16 and the display 22.

The term "display" relates to a monitor or projector that is configured to show graphic information like X-ray images, figures or instructions. The display 22 can be a wall-mounted arrangement or suspended from the ceiling. The display 22 can also be a head-worn display, for example for displaying of virtual reality or augmented reality.

In an option of Fig. 1, the distinctive feature of the device is at least one of the group comprising: a marker of the device, a tool of the device and a tip of the device.

The marker of a device can be used for finding the device within the image; the tip can be used for navigation purposes. The tool of a device can also be used for navigation purposes.

In an option of Fig. 1, for the computing of the secondary radiation characteristic, the data processor 14 is configured to choose a decreased radiation dose that results in a lower signal to noise ratio in the image portion comprising the at least one distinctive feature, which lower signal to noise ratio is above a predetermined threshold dependent on the distinctive feature.

The radiation dose is lowered as much as possible to find the dose that results in markers still being visible to a predetermined visibility degree. Basically, it is looked at the markers to identify to what dose the radiation can be lowered, in which the markers are still visible.

In an option of Fig. 1, for the computing of the secondary radiation characteristic, the data processor 14 is configured to assume a navigation path based on the device inside the vessel, and to assess an upcoming image area ahead for adapting the radiation dose for an upcoming imaging step.

For example, when the instrument is about to pass a vertebra structure, a higher dose may be selected to ensure that the image with the vertebra structure is actually usable. The goal is to have a good navigation image every time, but with just enough dose for navigating. Therefore, it is looked ahead in the image.

In an option of Fig. 1, for determining the signal to noise ratio of an image, the data processor 14 is configured to identify relevant structures in the image. The data processor 14 is configured to determine the average pixel intensity for these structures, and to measure noise as the standard deviation of pixel intensities in a background region without significant signal. The data processor 14 is configured to determine the signal to noise ratio by dividing the average signal by the standard deviation of the noise.

A higher signal to noise ratio indicates a clearer image. The signal to noise ratio increases with increasing X-ray dose. Furthermore, the signal to noise ratio can be determined by identifying the markers with a trained neural network and calculating as signal the average intensity of the pixels identified as belonging to the markers and the noise as the standard deviation of the intensities in the image not belonging to the markers and at >d pixels distance from any marker pixel.

In an option of Fig. 1, for determining whether the inserted device is within the predetermined region of the subject, the data input 12 is configured to provide location information of the predetermined region of the subject. The data processor 14 is configured to register the location information to a current X-ray image, and to identify the location of the device in the current X-ray image. The data processor 14 is configured to determine a spatial relation of the location of the device and the predetermined region of the subject.

In an option of Fig. 1, for identifying the distinctive feature in the X-ray image, the data processor 14 is configured to identify at least one of the group comprising i) an instrument classification, ii) a device-related data, and iii) a type of treatment.

In an option of Fig. 1, for providing location information, the data input 12 is configured to provide pre-operational image data or image data acquired during the current intervention.

In an example, the identifying of the treatment or subject area is done by determining structures that link to a current treatment setting. For instance, when a percutaneous coronary intervention is selected, the treatment or subject area of the subject is containing the heart with the coronary vessels. The algorithm determining the region of interest will then use an object detection algorithm, for instance YOLO, to detect whether the heart is in the image. If in the image, and the device is outside this area, then low dose X-ray is used; while, when inside, a normal X-ray dose is used. The term normal relates to a dose that results in image data in which more details are visible than on the lower dose images.

In an example, the region of interest is a rectangle within the X-ray image possibly derived from a cuboid defined on a pre-operational 3D scan. The rectangle or cuboid is the bounding box of the tissue of interest. This bounding box can be manually placed on the 2D or 3D image by the user; or it can be computed from any available tissue segmentation image; or it can be generated by a neural network that generates the ROI directly. In case of a 2D reference image, the spatial relationship between subsequent X-ray images is known. In case of 3D pre-op scans, a registration method is applied to align the X-ray images, e.g. by the user identifying landmarks in both the reference 2D and pre-op 3D scans, or by an automatic 2D-3D image registration.

In an example, for the computing of the secondary radiation characteristic for acquiring secondary X-ray images with the secondary radiation characteristic to generate images for further guidance of the inserted device, it is provided information about an application of a contrast agent.

In an option of Fig. 1, the data processor 14 is further configured to determine whether the inserted device is within a predetermined region of the subject. The data processor 14 is also configured to select to acquire X-ray images with: i) the secondary radiation characteristic if the inserted device is not within the predetermined region of interest; or with: ii) the primary radiation characteristic if the inserted device is within the predetermined region of interest.

In an option of Fig. 1, to determine the predetermined region of the subject in an image, the data processor 14 is configured to identify a subject area that contains a treatment area, and/or to identify a region of interest of the subject.

In an example, it is further provided: to determine the treatment area within the region of interest. It is further provided: to determine whether the inserted device is within the treatment area, and to acquire X-ray images with: i) the secondary radiation characteristic if the inserted device is not within the treatment area; or with: ii) the primary radiation characteristic if the inserted device is within the treatment area.

Fig. 2 shows an example of an X-ray image 50 of a region of interest of a subject. An anatomic structure 52 is shown, for example comprising ribs 54. An instrument 56 is inserted inside a vascular structure. As an example, the instrument 56 is a balloon catheter. The balloon catheter has two markers 58. The markers 58 provide a distinctive feature identifiable in the X-ray image 50. Further, a tip 60 can be seen in the X-ray image 50.

The X-ray image 50 is acquired with a primary radiation characteristic and a signal to noise ratio relating to an image portion comprising the at least one distinctive feature like the two markers can now be determined. A secondary radiation characteristic for acquiring secondary X-ray images with the secondary radiation characteristic for further guidance of the inserted device can now be determined. It is then either selected the primary radiation characteristic or the secondary radiation characteristic for acquiring further X-ray images. This is depending on the current location within subject or the current treatment phase, or both. If the user, i.e. the clinician, is within a steering procedure, the secondary radiation characteristic can be applied to save dose. If the user, i.e. the clinician, is within or is about to begin an actual treatment procedure, the primary radiation characteristic can be applied to provide optimum image information. Thus, an optimized application of radiation dose is provided that serves the particular task in an adapted manner.

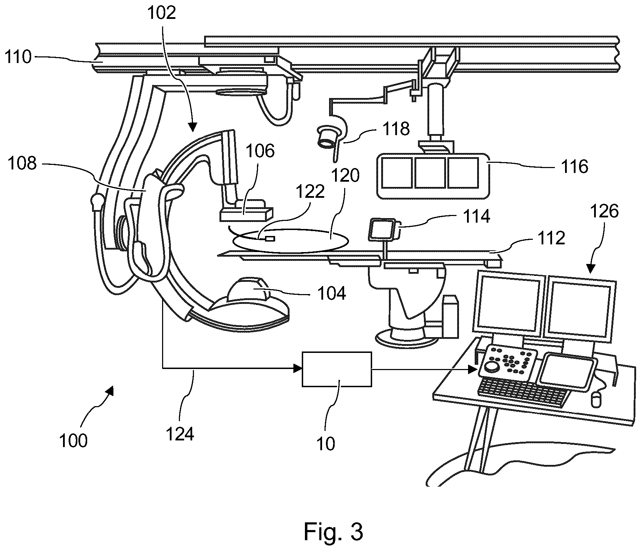

Fig. 3 shows an example of a medical X-ray imaging system 100 for intraluminal device guidance. The medical X-ray imaging system 100 comprises an X-ray imaging device 102 and an example of the control device 10 according to one of the preceding examples. The X-ray imaging device 102 is configured to provide the X-ray image 50 of the vascular structure with an inserted device having at least one distinctive feature identifiable in the X-ray image 50. The control device 10 is configured to provide the selected radiation characteristic to the X-ray imaging device to acquire further X-ray images.

In Fig. 3, the X-ray imaging arrangement 102 is shown as a C-arm device. The X-ray imaging arrangement 102 comprises an X-ray source 104 and an X-ray detector 106 mounted to opposing ends of a movable C-arm arrangement 108 that is suspended from a ceiling rail system 110. Further, a subject support 112 is shown with a bedside controller interface 114. Still further, a display arrangement 116 and some lighting equipment 118 can be provided. A subject 120, e.g. a patient, is arranged on the subject support 112. In Fig. 3, as an option, the system 100 further comprises a ballon catheter 122 or guidewire for insertion into a vascular structure of the subject. In Fig. 3, the catheter 122 is inserted. Further components like an operating handle of the catheter or guidewire are provided, but not shown. The X-ray imaging arrangement 102 is data-connected to the device 10 for indicating balloon expansion by a data-connection, which can be wire-bound or wireless. The data-connection is indicated with connection line 124. As an option, Fig. 3 shows a console 126 in the right foreground that may comprise interface components like a display, keyboard, mouse, trackpad, control knobs and the like. The console is provided for operating and controlling the various equipment.

In an option of Fig. 3, the control device 10 is configured to provide the secondary radiation characteristic to the X-ray imaging device to acquire secondary X-ray image data with the secondary radiation characteristic.

In another option of Fig. 3, the system further comprises a display, e.g. the display 22 or the display arrangement 116, configured to provide navigation information based on the secondary X-ray image data.

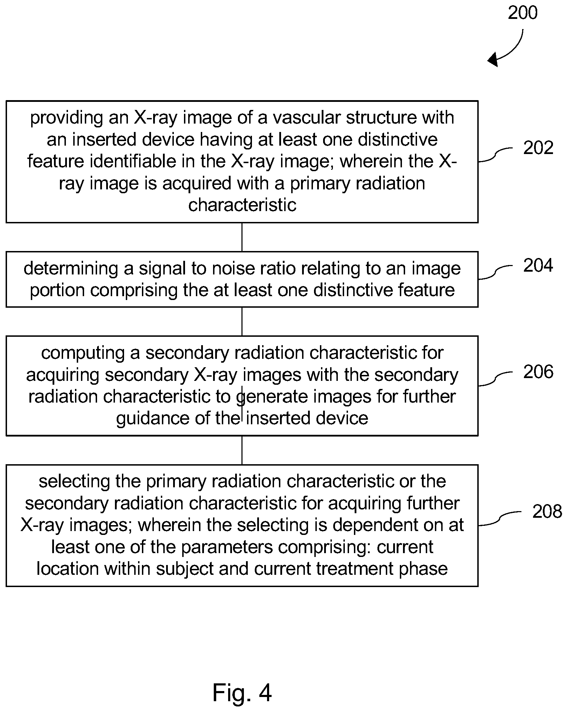

Fig. 4 shows basic steps of an example of a method 200 for intraluminal device guidance with minimized X-ray dose. The method 200 comprises the following steps:
- In a first step 202, an X-ray image of a vascular structure with an inserted device is provided having at least one distinctive feature identifiable in the X-ray image. The X-ray image is acquired with a primary radiation characteristic.
- In a second step 204, a signal to noise ratio is determined relating to an image portion comprising the at least one distinctive feature.
- In a third step 206, a secondary radiation characteristic is computed for acquiring secondary X-ray images with the secondary radiation characteristic to generate images for further guidance of the inserted device.
- In a fourth step 208, the primary radiation characteristic or the secondary radiation characteristic is selected for acquiring further X-ray images. The selecting is dependent on at least one of the parameters comprising: current location within subject and current treatment phase.

In an example of the method, it is further provided: acquiring secondary X-ray image data with the secondary radiation characteristic; and providing navigation information based on the secondary X-ray image data.

In an example of the method, for the computing of the secondary radiation characteristic, a decreased radiation dose is chosen that results in a lower signal to noise ratio in the image portion comprising the at least one distinctive feature, which lower signal to noise ratio is above a predetermined threshold dependent on the distinctive feature.

In an example of the method, for the computing of the secondary radiation characteristic, a navigation path is assumed based on the device inside the vessel, and an upcoming image area ahead is assessed for adapting the radiation dose for an upcoming imaging step.

In an example of the method, for determining the signal to noise ratio of an image, it is provided the steps of: identifying relevant structures in the image; determining the average pixel intensity for these structures; measuring noise as the standard deviation of pixel intensities in a background region without significant signal; and determining the signal to noise ratio by dividing the average signal by the standard deviation of the noise.

In an example of the method, it is further provided: determining whether the inserted device is within a predetermined region of the subject; and acquiring X-ray images: i) with the secondary radiation characteristic if the inserted device is not within the predetermined region of interest; or ii) with the primary radiation characteristic if the inserted device is within the predetermined region of interest.

In an example of the method, for determining whether the inserted device is within the predetermined region of the subject, it is provided the steps of: providing location information of the predetermined region of the subject and registering the location information to a current X-ray image; identifying the location of the device in the current X-ray image; and determining a spatial relation of the location of the device and the predetermined region of the subject.

In an option, a computer program is provided that comprises instructions which, when the program is executed by a computer, cause the computer to carry out the method of one the preceding examples.

In an example, a program element for controlling an apparatus according to one of the examples above is provided, which program element, when being executed by a processing unit, is adapted to perform the method steps of one of the method examples above.

In an example, a computer readable medium having stored the computer program of the preceding example is provided.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. **In** particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A control device (10) for intraluminal device guidance with minimized X-ray dose, the device comprising:
- a data input (12);
- a data processor (14); and
- an output interface (16);
wherein the data input is configured: to provide an X-ray image (18) of a vascular structure with an inserted device having at least one distinctive feature identifiable in the X-ray image; wherein the X-ray image is acquired with a primary radiation characteristic;
wherein the data processor is configured: to determine a signal to noise ratio relating to an image portion comprising the at least one distinctive feature; to compute a secondary radiation characteristic for acquiring secondary X-ray images with the secondary radiation characteristic to generate images for further guidance of the inserted device; and to select the primary radiation characteristic or the secondary radiation characteristic for acquiring further X-ray images; wherein the selecting is dependent on at least one of the parameters comprising: current location within subject and current treatment phase; and
wherein the output interface is configured: to provide the selected radiation characteristic for acquiring further X-ray images.

2. Device according to claim 1, wherein the distinctive feature of the device is at least one of the group comprising:
- marker of the device;
- tool of the device; and
- tip of the device.

3. Device according to claim 1 or 2, wherein, for the computing of the secondary radiation characteristic, the data processor is configured to choose a decreased radiation dose that results in a lower signal to noise ratio in the image portion comprising the at least one distinctive feature, which lower signal to noise ratio is above a predetermined threshold dependent on the distinctive feature.

4. Device according to claim 1, 2 or 3, wherein, for the computing of the secondary radiation characteristic, the data processor is configured to assume a navigation path based on the device inside the vessel, and to assess an upcoming image area ahead for adapting the radiation dose for an upcoming imaging step.

5. Device according to one of the preceding claims, wherein, for determining the signal to noise ratio of an image, the data processor is configured: to identify relevant structures in the image; to determine the average pixel intensity for these structures; to measure noise as the standard deviation of pixel intensities in a background region without significant signal; to determine the signal to noise ratio by dividing the average signal by the standard deviation of the noise.

6. Device according to one of the preceding claims, wherein, for determining whether the inserted device is within the predetermined region of the subject, the data input is configured: to provide location information of the predetermined region of the subject; and
wherein the data processor is configured: to register the location information to a current X-ray image; to identify the location of the device in the current X-ray image; and to determine a spatial relation of the location of the device and the predetermined region of the subject.

7. Device according to one of the preceding claims, wherein, for identifying the distinctive feature in the X-ray image, the data processor is configured: to identify at least one of the group comprising:
i) instrument classification;
ii) device-related data; and
iii) type of treatment.

8. Device according to one of the preceding claims, wherein, for providing location information, the data input is configured: to provide pre-operational image data or image data acquired during the current intervention.

9. Device according to one of the preceding claims, wherein the data processor is further configured: to determine whether the inserted device is within a predetermined region of the subject; and to select to acquire X-ray images with: i) the secondary radiation characteristic if the inserted device is not within the predetermined region of interest; or with: ii) the primary radiation characteristic if the inserted device is within the predetermined region of interest.

10. Device according to claim 9, wherein, to determine the predetermined region of the subject in an image, the data processor is configured: to identify a subject area that contains a treatment area; and/or to identify a region of interest of the subject.

11. A medical X-ray imaging system (100) for intraluminal device guidance, the system comprising:
- an X-ray imaging device (102); and
- a control device (10) according to one of the preceding claims;
wherein the X-ray imaging device is configured to provide the X-ray image of the vascular structure with an inserted device having at least one distinctive feature identifiable in the X-ray image; and
wherein the control device is configured to provide the selected radiation characteristic to the X-ray imaging device to acquire further X-ray images.

12. System according to claim 11, wherein the control device is configured to provide the secondary radiation characteristic to the X-ray imaging device to acquire secondary X-ray image data with the secondary radiation characteristic.

13. System according to claim 11 or 12, further comprising a display (116) configured to provide navigation information based on the secondary X-ray image data.

14. A method (200) for intraluminal device guidance with minimized X-ray dose, the method comprising the following steps:
- providing (202) an X-ray image of a vascular structure with an inserted device having at least one distinctive feature identifiable in the X-ray image; wherein the X-ray image is acquired with a primary radiation characteristic;
- determining (204) a signal to noise ratio relating to an image portion comprising the at least one distinctive feature;
- computing (206) a secondary radiation characteristic for acquiring secondary X-ray images with the secondary radiation characteristic to generate images for further guidance of the inserted device; and
- selecting (208) the primary radiation characteristic or the secondary radiation characteristic for acquiring further X-ray images; wherein the selecting is dependent on at least one of the parameters comprising: current location within subject and current treatment phase.

15. Computer program comprising instructions which, when the program is executed by a computer, cause the computer to carry out the method of claim 14.
